# EUROPEAN PATENT SPECIFICATION

(11) **EP 0 680 296 B1**
(45) Date of publication and mention of the grant of the patent: **14.05.1997**
(21) Application number: 93906459.8
(22) Date of filing: 03.03.1993
(51) Int. Cl.: A61F 5/445, A61F 5/44

(54) **A COLLECTION BAG FOR URINE FROM URINE-INCONTINENT AND UROSTOMY-OPERATED HUMANS AS WELL AS AN OUTLET VALVE SYSTEM THEREFOR**
URINAUFFANGBEUTEL SOWIE AUSLASSVENTILSYSTEM DAFÜR FÜR URININKONTINENZ LEIDENDE ODER UROSTOMIE-OPERIERTE PERSONEN
SAC COLLECTOR D'URINE ET ENSEMBLE VALVE DE VIDANGE POUR PERSONNES SOUFFRANT D'INCONTINENCE URINAIRE OU AYANT SUBI UNE UROSTOMIE

(30) Priority: 04.03.1992 DK 290/92
(43) Date of publication of application: 08.11.1995
(73) Proprietor: COLOPLAST A/S, DK-3060 Espergaerde (DK)
(72) Inventor: ANDERSEN, Henrik, Bork, DK-2400 Copenhagen NV (DK)
(74) Representative: Christiansen, Ejvind
(86) International application number: DK9300076
(87) International publication number: WO9317642

(56) References cited:
- DE-C- 3 039 859
- NO-B- 169 154
- US-A- 4 300 560

## Description

The invention concerns a collection bag for urine from urine-incontinent or urostomy-operated humans as well as an outlet valve system for such a collection bag.

Such bags are known and typically consist of two layers of plastic sheet which are joined by welding along their peripheries to form a cavity between the sheets for collection of urine. For the discharge of the bag it has at its lowermost part an outlet valve system which the user can open so that the collected urine runs out of the bag. Discharge from such a valve system always takes place through a hose or tube member which is connected with the closing mechanism of the valve system and has a discharge opening. Following emptying of the bag at least a few drops of urine will always be left in the discharge hose or tube member, and to prevent malodours from such drops it is necessary also to close the discharge opening of the discharge hose or tube member to prevent the remaining drops from getting into contact with the clothes with the consequent air nuisances. The discharge opening of the discharge hose or tube member is usually closed by plugging this opening. Separate movements are required for the closing of the two closing mechanisms of the valve system, and the mounting of the plug in the discharge opening moreover requires a certain dexterity which cannot always be expected of users of such equipment. Valve systems with the drawbacks mentioned here are described e.g. in US Patent Specification 4 300 560 and in DK Patent Application 3743/84 which has been laid open to public inspection.

The object of the invention is to provide an outlet valve system which is easier and safer to operate than the known valve systems. This is obtained by an outlet valve system according to the invention which is of the type defined in the introductory portion of claim 1 and which is characterized by the features defined in the characterizing portion of claim 1. Since both closing mechanisms of the valve system are operated at the same time and by one and same movement of one and the same movable means, the operation is simplified signicantly, while complete closing of both closing mechanisms is always ensured. Furthermore, in contrast to the known valve systems, the valve system of the invention can be operated by one hand.

Another object of the invention is to provide a collection bag which is easy to operate. Such a bag is defined in claim 10. In known urine collection bags the discharge tube and the valve system for closing and blocking said tube are usually welded in the bottom weld of the bag, and thus have parts protruding below the periphery of the bag. These parts often contact the user's skin. Since the discharge tube and the valve system parts are usually made of a material having a greater hardness and/or thickness than the sheets of the collection bag and may moreover have edges or even burrs, the contact between these parts and the user's skin is often inconvenient to the user.

A preferred embodiment of the collection bag of the invention provides a collection bag which is not vitiated by the drawbacks described above. This preferred embodiment is defined in claim 11. When the valve system is attached to that one of the sheets which faces away from the user in the position of use so that it is positioned completely within the periphery of the sheets in its closed state, then valve system contact with the user's skin will be obviated completely.

The invention will be described more fully below with reference to the drawing, in which
fig. 1 shows a collection bag according to the invention provided with an outlet valve system according to the invention for use by urostomy-operated humans,
fig. 2 shows a collection bag according to the invention provided with an outlet valve system according to the invention for use by urine-incontinent humans,
fig. 3 shows a section through the preferred embodiment of the valve system in fig. 1 which is in its third position along the line III-III,
fig. 4 shows a section through the valve system in fig. 3 along the line IV-IV,
fig. 5 shows the valve system in figs. 3 and 4 in its first position,
fig. 6 shows the valve system in fig. 3 along the line VI-VI,
fig. 7 shows a second embodiment of the valve system according to the invention in which the movable means is linearly displacable with respect to the base member, shown from the side in its third position,
fig. 8 is a sectional view of the valve system of fig. 7 in its second position, and
fig. 9 is a sectional view of the valve system of fig. 7 in its first position.

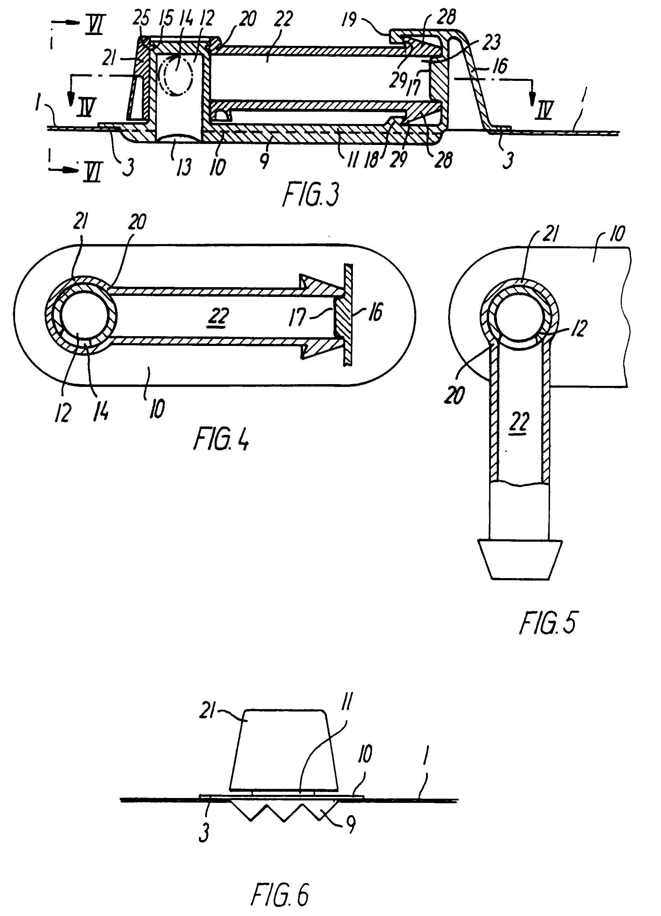

The valve system of figs. 3 and 6 has a base member (10) which is secured along its entire periphery by means of a weld (3) to a first sheet (1) of the bag. The base member 10 of the valve system has a relatively stiff base part (11) with a cylindrical portion (12) which protrudes from the base part (11). The cylindrical portion (12) has a cavity (13) which is closed at one end and has an opposite end which opens into the cavity of the bag. The cylindrical side face of the cylindrical portion (12) has a through hole (14) into the cavity (13).

The valve system moreover has a movable means (20) which is formed by a discharge tube member having an inlet section (21), a straight tube section (22) and a discharge opening (23), the inlet section (21) of the discharge tube member surrounding the cylindrical portion (12) of the first member. The cylindrical portion (12) and the inlet section (21) are intimately engaged at least along parts of their cylindrical faces which are in mutual contact. An annular rib (25) on the inlet section (21) engages a corresponding annular groove (15) on the cylindrical portion (12). The rib (25) and the groove (15) serve partly as a seal, partly as a closing mechanism against separation.

The base part (11) has some protruding ribs (9) on the side facing away from the cylindrical portion (12). In the shown embodiment the ribs (9) extend along the base part (11). However, these ribs may equally well extend transversely or obliquely, or, instead of ribs, there may just be some protruding knobs or areas. The ribs (9) serve two purposes. One is to contribute to stiffening the base part (11), another is to provide the spacers between the two bag walls (1, 2) (see in particular fig. 6), thus making it easier to get the last drops out of the collection bag when said bag is emptied. In particular the latter object is important, and, as mentioned before, this purpose may equally be served by providing the base part (11) with protruding knobs or areas.

Figs. 4 and 5 show how the movable means (20) with the inlet section (21) of the valve system can rotate about the cylindrical portion (12) of the first member. In fig. 5 the movable means (20) has been rotated to a position in which the straight tube section (22) of the means is arranged concentrically opposite the hole (14) in the cylindrical portion (12) of the base part, and the valve system is thus in its first position. In fig. 4 the movable means of the valve system is arranged in another angular position with respect to the base member (10) of the valve system. The shown tube section (22) has here been rotated away from the hole (14), and the inlet section (21) is sealing engaged with the cylindrical portion (12). In the second position (not shown) of the valve system the movable means (20) is arranged in an angular position between the positions shown in figs. 4 and 5.

The base member (10) of the valve system moreover has a cover (16) which protrudes from the base part (11). The cover (16) has a projection (17) which extends into the discharge opening (23) of the discharge tube member in the third position of the valve system, which is shown in figs. 3 and 4. The part (16) with the projection (17) is shaped such as to sealingly engage the discharge opening (23) of the discharge tube member. The valve system is made of a polymeric material, preferably a semi-rigid material such as e.g. polyethylene. This provides the further advantage that the projection (17) together with the discharge opening (23) serves as a snap lock for the closed state of the valve system.

It will be seen from fig. 3 that the base part (11) has a projection or a hook (18), and that the cover (16) moreover has a resilient hook (19). Near its discharge opening (23) the tube section (22) has a conical portion (28) with an edge (29) which engages the hooks (18, 19) in the third position of the valve system. When engaged with the edge (29) the hooks (18, 19) ensure that the discharge opening (23) is correctly engaged with the projection (17), and in particular that the movable means (20) can be moved from the third position only by rotation about the cylindrical portion (12). When the collection bag is filled completely, the sheet (1), to which the valve system is secured, will tend to bend, but when the hooks (18, 19) are engaged with the edge (29), the valve system is protected against unintentional opening or leaks, because the movable means (20) is secured against such a movement.

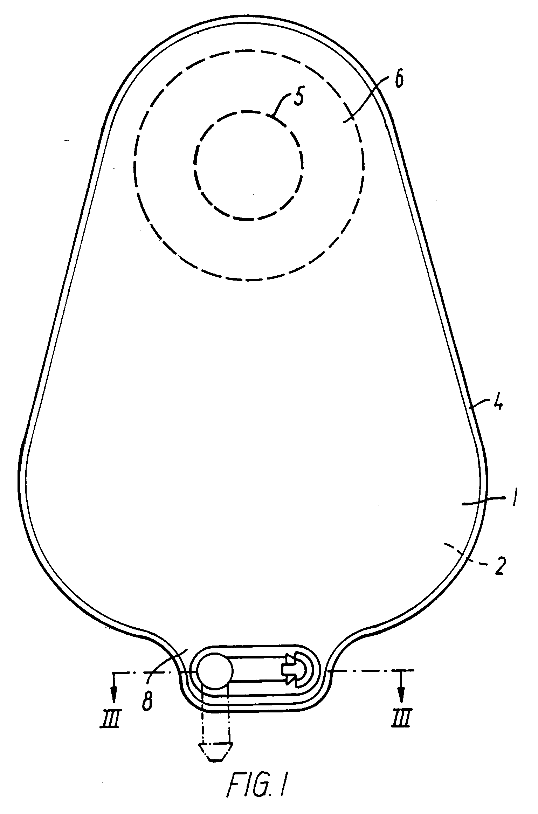

The preferred embodiment of a valve system according to the invention shown in figs. 3-6 has the further advantage that in its first position, when mounted on a collection bag as shown in fig. 1, it is not in contact with the user's skin.

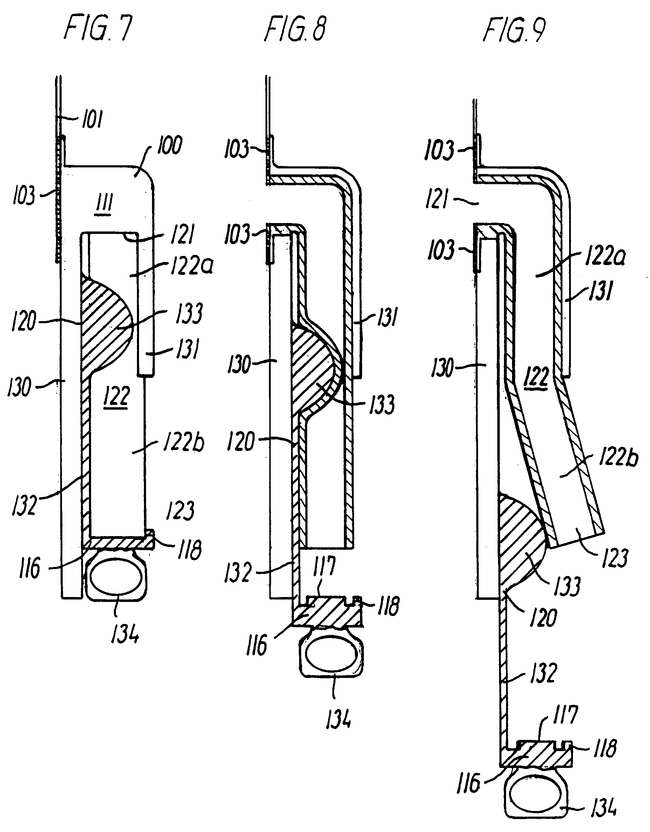

Figs. 7, 8 and 9 show a second embodiment of the valve system of the invention in a third position, a second position and a first position, respectively. The valve system has a base member (100) which is secured by means of an annular weld (103) to a first sheet (101) in a collection bag around an opening in this sheet. The base member (100) has two relatively stiff rails (130, 131) which are substantially parallel with the first sheet (101) of the collection bag. The long rail (130) and the short rail (131) are connected with a housing (111) which simultaneously surrounds the inlet section (121) in a tube member (122). The inlet section (121) may e.g. be welded to the housing (111) or be firmly connected with the housing (111) in another manner. The inlet section (121) of the tube member communicates with the opening of the sheet (101). The tube member (122) is relatively flexible and is retained in its upper portion (122a) between the two rails (130, 131). The lower portion (122b) of the tube member is clear of the short rail (131). The tube member moreover has a discharge opening (123).

The movable means (120) is formed by a relatively stiff rail (132) which can be displaced in the space between the long rail (130) and the tube member (122). In its upper end the rail (132) has a protruding body (133) which faces the tube member (122) and, in the second and third positions of the valve system, squeezes the tube member (122) together and thus prevents flow therethrough. In its lower end the rail (132) has a protruding cover (116) with a projection (117), a hook (118) and a finger grip (134). When the valve system is in its third position (fig. 7), the cover (116) sealingly engages the discharge opening (123), the projection (117) simultaneously engaging the inner periphery of the discharge opening.

The movable means (120) can be displaced in its three positions by means of the finger grip (134).

In its first position (fig. 9) the lower portion (122b) of the tube member bends outwardly, and liquid can freely run out of the collection bag, into to the inlet section (121) through the tube member (122) and out of the discharge opening (123).

In its second position (fig. 8) the tube member (122) is squeezed together between the protruding body (133) and the short rail (131), thereby preventing additional liquid to run down into the lower portion (122b) of the tube member. The drops present in the lower portion (122b) of the tube member can still pass out through the discharge opening (123).

In its third position (fig. 7) the tube member (122) is likewise squeezed together between the body (133) and the short rail, but the cover (116) simultaneously sealingly engages the discharge opening (123).

Fig. 1 shows a collection bag for use by urostomy-operated humans with an opening (5). Around the opening (5) the bag has an adhesive layer (6) of a skin-friendly adhesive material, e.g. as described in the Applicant's DK Patent Specifications 147 034 and 147 035 (correspond to US Patent Specifications 4 231 369 and 4 367 632), by means of which the urostomy bag can be adhered in a manner known per se to the user's skin with the opening (5) around the stoma of the user.

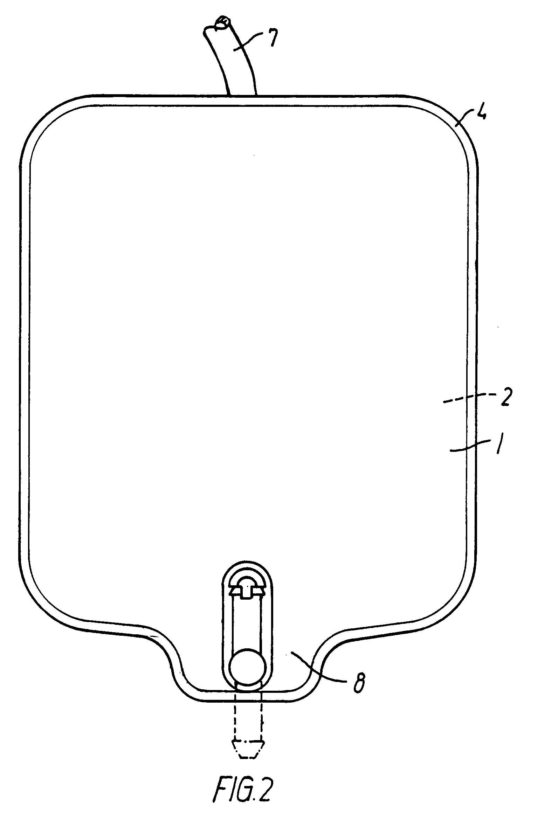

Fig. 2 shows a collection bag for use by urine-incontinent humans with an inlet tube (7). The collection bag may e.g. be constructed as a leg bag and have the attachment means necessary for this.

The collection bags of the invention may advantageously be provided with internal sheet members or intermediate walls, e.g. like the collection bag described in the Applicant's Danish patent application having the same filing date as the present DK application.

The bags in figs. 1 and 2 consist of two layers of plastic sheet (1, 2) which are joined along their common pheriphery, e.g. by welding or glueing (4), to form the two sheet walls (1, 2) of the collection bag. The two bags shown both have a constriction or a so-called sump (8) at their lower end. The bags of the invention may be constructed without such a sump (8). However, it is preferred that the bags have a sump (8), because such a sump (8) contributes to collecting the liquid at the discharge opening and thus makes it easier to get the last drops out of the bag when it is emptied. Each of the bags has a preferred outlet valve system according to the invention, as described above, secured to the sheet wall (1) at the sump (8) in the same manner as explained in connection figs. 3 and 6. The outlet valve systems are shown in the closed state (in their third position) in figs. 1 and 2, and it is noted that in both cases the valve systems are positioned entirely within the peripheries of the respective bags. The sheet wall (1), to which the valve system is secured, faces away from the user's skin in the situation of use, and the valve system will therefore not contact the user's skin, because the bag is always positioned between the valve system and the user's skin.

As mentioned before, fig. 6 shows that the base part (11) of the valve system is provided with ribs (9). The ribs (9) extend in the entire length of the base part (11). The rear sheet (2) of the bag is always kept spaced from the valve system by means of the ribs (9) so that there is always free discharge from the interior of the bag to the hole (13) in the valve system.

## Claims

1. An outlet valve system for a collection bag for urine from urine-incontinent or urostomy-operated humans, consisting of a base member (10, 100) and at least one movable means (20, 120) which together form a discharge tube or hose member with an inlet section (21, 121) and a discharge opening (23, 123), the inlet section of the discharge tube or hose member being in contact with an opening for leading into the cavity of the bag, a stop valve designating a valve for the opening for leading into the cavity of the bag as well as a closing valve designating a valve for the discharge opening, said valve system having three positions, a first position in which both the stop valve and the closing valve are open, a second position in which the stop valve is closed and the closing valve is open, and a third position in which both valves are closed, **characterized** in that the movable means (20, 120) is rotatably and/or linearly displacable with respect to the base member and by means of which movable means the valve system can be set in the three positions.

2. A valve system according to claim 1, **characterized** in that the movable means (20) is rotatably displacable with respect to the base member (10).

3. A valve system according to claim 1, **characterized** in that the movable means (120) is linearly displacable with respect to the base member (100).

4. A valve system according to claims 1-3, **characterized** in that the base member (10, 100) or the displacable means (20, 120) comprises a cover (16, 116) which sealingly engages the discharge opening (23, 123) of the discharge tube member in the third position of the valve system.

5. A valve system according to claim 4, **characterized** in that the cover (16, 116) has a face which is complementary to the discharge opening (23, 123) of the discharge tube member, preferably in that the cover has a projection (17, 117) which protrudes into the discharge opening (23, 123) and sealing engages it when the valve system is in its third position.

6. A valve system according to claims 1-5, **characterized** in that the movable means (20) is formed by the discharge tube member (21, 22, 23).

7. A valve system according to claims 1-6, **characterized** in that the base member (10, 100) and the movable means (20, 120) have respective cooperating locking means (18, 19, 29) which are in engagement when the valve system is in its third position.

8. A valve system according to claim 7, **characterized** in that the locking means is constructed as projections (29) and hooks (18, 19), respectively, arranged in a plane, which, with respect to the movable part, is perpendicular to the direction of the displacement of said part.

9. A valve system according to claims 1-8, **characterized** in that the side of the base member (10, 100) facing the collection bag sheet (1) is formed with protruding knobs or grooves (9) in particular longitudinal grooves which preferably extend into the bag through one bag sheet wall (1) to form spacers between the two bag sheet walls (1, 2).

10. A collection bag for collection of urine from urine-incontinent or urostomy-operated humans, comprising two sheets (1, 2) which are joined along their peripheries and define a cavity, and a valve system consisting of a base member (10, 100) and at least one movable means (20, 120) which together form a discharge tube or hose member (22, 122) with an inlet section (21, 121) and a discharge opening, the inlet section (21, 121) of the discharge tube or hose member being in contact with an opening for leading into the cavity of the bag, a stop valve designating a valve for the opening for leading into the cavity of the bag as well as a closing valve designating a valve for the discharge opening, said valve system having three positions, a first position in which both the stop valve and the closing valve are open, a second position in which the stop valve is closed and the closing valve is open, and a third position in which both valves are closed, **characterized** in that the movable means (20, 120) is rotatably and/or linearly displacable with respect to the base member and by means of which movable means the valve system can be set in the three positions.

11. A collection bag according to claim 10, **characterized** in that the valve system in its third position is positioned entirely within the peripheries of the sheets (1, 2).

## Patentansprüche

1. Auslaßventilsystem für einen Urinauffangbeutel für urininkontinente oder an den Harnableitungswegen operierte Personen, das aus folgendem besteht:
einem Grundelement (10, 100) und zumindest einer beweglichen Einrichtung (20, 200), die zusammen ein Auslaßrohr oder ein Schlauchelement mit einem Einlaßabschnitt (21, 121) und einer Auslaßöffnung (23, 123) bilden, wobei der Einlaßabschnitt des Auslaßrohrs oder des Schlauchelementes mit einer Öffnung in Kontakt ist, die in den Hohlraum des Beutels führt, sowohl einem Halteventil, mit dem ein Ventil für die in den Hohlraum des Beutels führende Öffnung bezeichnet ist, als auch einem Schließventil, mit dem ein Ventil für die Auslaßöffnung bezeichnet ist, wobei das Ventilsystem drei Positionen hat, eine erste Position, bei der sowohl das Halteventil als auch das Schließventil geöffnet sind, eine zweite Position, bei der das Halteventil geschlossen ist und das Schließventil geöffnet ist, und eine dritte Position, bei der beide Ventile geschlossen sind,
**dadurch gekennzeichnet, daß**
die bewegliche Einrichtung (20, 120) gegenüber dem Grundelement drehend und/oder geradlinig versetzbar ist und das Ventilsystem mittels der beweglichen Einrichtung in die drei Positionen gesetzt werden kann.

2. Ventilsystem nach Anspruch 1,
**dadurch gekennzeichnet, daß**
die bewegliche Einrichtung (20) gegenüber dem Grundelement (10) drehend versetzbar ist.

3. Ventilsystem nach Anspruch 1,
**dadurch gekennzeichnet, daß**
die bewegliche Einrichtung (120) gegenüber dem Grundelement (100) geradlinig versetzbar ist.

4. Ventilsystem nach den Ansprüchen 1-3,
**dadurch gekennzeichnet, daß**
das Grundelement (10, 100) oder die versetzbare Einrichtung (20, 120) eine Abdeckung (16, 116) aufweist, die mit der Auslaßöffnung (23, 123) des Auslaßrohrelementes in der dritten Position des Ventilsystems in dichtem Eingriff ist.

5. Ventilsystem nach Anspruch 4,
**dadurch gekennzeichnet, daß**
die Abdeckung (16, 116) eine Fläche hat, die der Auslaßöffnung (23, 123) des Auslaßrohrelementes komplementär ist, indem die Abdeckung vorzugsweise einen Vorsprung (17, 117) hat, der in die Auslaßöffnung (23, 123) hineinragt und mit ihr in dichtem Eingriff ist, wenn das Ventilsystem in seiner dritten Position ist.

6. Ventilsystem nach den Ansprüchen 1-5,
**dadurch gekennzeichnet, daß**
die bewegliche Einrichtung (20) durch das Auslaßrohrelement (21, 22, 23) gebildet ist.

7. Ventilsystem nach den Ansprüchen 1-6,
**dadurch gekennzeichnet, daß**
das Grundelement (10, 100) und die bewegliche Einrichtung (20, 120) entsprechende zusammenwirkende Sperreinrichtungen (18, 19, 29) haben, die im Eingriff sind, wenn das Ventilsystem in seiner dritten Position ist.

8. Ventilsystem nach Anspruch 7,
**dadurch gekennzeichnet, daß**
die Sperreinrichtung so aufgebaut ist, daß Vorsprünge (29) und Haken (18, 19) jeweils in einer Ebene angeordnet sind, die in bezug auf das bewegliche Teil senkrecht zu der Versetzrichtung des Teiles ist.

9. Ventilsystem nach den Ansprüchen 1-8,
**dadurch gekennzeichnet, daß**
die Seitenfläche des Grundelementes (10, 100), die der Auffangbeutelfolie (1) zugewandt ist, mit vorstehenden Nasen oder Nuten (9) ausgebildet ist, insbesondere mit Längsnuten, die vorzugsweise durch eine Beutelfolienwand (1) in den Beutel hinein reichen, um Abstandhalter zwischen den beiden Beutelfolienwänden (1, 2) zu bilden.

10. Auffangbeutel zum Auffangen von Urin von urininkontinenten oder an den Harnableitungswegen operierten Personen, der zwei Folien (1, 2), die entlang ihrer Ränder verbunden sind und einen Hohlraum definieren, und ein Ventilsystem aufweist, das aus folgendem besteht:
einem Grundelement (10, 100) und zumindest einer beweglichen Einrichtung (20, 200), die zusammen ein Auslaßrohr oder ein Schlauchelement mit einem Einlaßabschnitt (21, 121) und einer Auslaßöffnung (23, 123) bilden, wobei der Einlaßabschnitt des Auslaßrohrs oder des Schlauchelementes mit einer Öffnung in Kontakt ist, die in den Hohlraum des Beutels führt, sowohl einem Halteventil, mit dem ein Ventil für die in den Hohlraum des Beutels führende Öffnung bezeichnet ist, als auch einem Schließventil, mit dem ein Ventil für die Auslaßöffnung bezeichnet ist, wobei das Ventilsystem drei Positionen hat, eine erste Position, bei der sowohl das Halteventil als auch das Schließventil geöffnet sind, eine zweite Position, bei der das Halteventil geschlossen ist und das Schließventil geöffnet ist, und eine dritte Position, bei der beide Ventile geschlossen sind,
**dadurch gekennzeichnet, daß**
die bewegliche Einrichtung (20, 120) gegenüber dem Grundelement drehend und/oder geradlinig versetzbar ist und das Ventilsystem mittels der beweglichen Einrichtung in die drei Positionen gesetzt werden kann.

11. Auffangbeutel nach Anspruch 10,
**dadurch gekennzeichnet, daß**
das Ventilsystem in seiner dritten Position vollständig innerhalb der Ränder der Folien (1, 2) angeordnet ist.

## Revendications

1. Ensemble de valve de vidange pour un sac collecteur d'urine pour personnes souffrant d'incontinence urinaire ou ayant subi une urostomie, constitué d'une base (10, 100) et d'au moins un moyen mobile (20, 120), qui ensemble forment un tube ou tuyau de décharge avec une section d'entrée (21, 121) et une ouverture de décharge (23, 123), la section d'entrée du tube ou tuyau de décharge étant en contact avec une ouverture débouchant dans la cavité du sac, une valve d'arrêt formant une valve d'admission conduisant dans la cavité du sac, ainsi qu'une valve de fermeture formant une valve d'évacuation pour l'ouverture de décharge, ledit ensemble pouvant prendre trois positions, une première dans laquelle la valve d'arrêt et la valve de fermeture sont toutes les deux ouvertes, une deuxième dans laquelle la valve d'arrêt est fermée et la valve de fermeture est ouverte, et une troisième dans laquelle les deux valves sont fermées, caractérisé en ce que le moyen mobile (20, 120) est déplaçable par rotation et/ou linéairement par rapport à la base, cet ensemble pouvant être ajusté dans les trois positions grâce à ce moyen.

2. Ensemble de valve selon la revendication 1, caractérisé en ce que le moyen mobile (20) est déplaçable par rotation par rapport à la base (10).

3. Ensemble de valve selon la revendication 1, caractérisé en ce que le moyen mobile (120) est déplaçable linéairement par rapport à la base (100).

4. Ensemble de valve selon les revendications 1-3, caractérisé en ce que la base (10, 100) ou le moyen mobile (20, 120) comprend un bouchon (16, 116), qui engage de manière étanche l'ouverture de décharge (23, 123) du tube dans la troisième position.

5. Ensemble de valve selon la revendication 4, caractérisé en ce que le bouchon (16, 116) a une face complémentaire de l'ouverture de décharge (23, 123) du tube, de préférence en ce que le bouchon présente une saillie (17, 117) qui fait saillie dans l'ouverture (23, 123) et coopère avec elle de manière étanche lorsque l'ensemble est dans sa troisième position.

6. Ensemble de valve selon les revendications 1-5, caractérisé en ce que le moyen mobile (20) est formé par le tube de décharge (21, 22, 23).

7. Ensemble de valve selon les revendications 1-6, caractérisé en ce que la base (10, 100) et le moyen mobile (20, 120) comportent des éléments de verrouillage coopérants respectifs (18, 19, 29), engagés mutuellement lorsque l'ensemble est dans sa troisième position.

8. Ensemble de valve selon la revendication 7, caractérisé en ce que les éléments de verrouillage sont réalisés sous la forme de saillies (29) et de crochets (18, 19) respectivement, disposés dans un plan, qui, par rapport à la partie mobile, est perpendiculaire à la direction du déplacement de cette partie.

9. Ensemble de valve selon les revendications 1-8, caractérisé en ce que le côté de la base (10, 100) faisant face à la paroi du sac collecteur (1) est formée avec des renflements saillants ou des renfoncements (9), en particulier des renfoncements longitudinaux qui s'étendent de préférence dans le sac à travers sa paroi (1) pour former des organes d'espacement entre ses deux parois (1, 2).

10. Sac collecteur pour collecter l'urine de personnes souffrant d'incontinence urinaire ou ayant subi une urostomie, comprenant deux parois (1, 2) en feuille, jointes le long de leur périphérie et définissent une cavité, et un ensemble de valve, constitué d'une base (10, 100) et d'au moins un moyen mobile (20, 120), qui forment ensemble un tube ou tuyau de décharge (22, 122) avec une section d'entrée (21, 121) et une ouverture de décharge, la section d'entrée (21, 121) du tube ou tuyau de décharge étant en contact avec une ouverture débouchant dans la cavité du sac, une valve d'arrêt formant une valve d'admission conduisant dans la cavité du sac, ainsi qu'une valve de fermeture formant une valve d'évacuation pour l'ouverture de décharge, ledit ensemble de valve pouvant prendre trois positions, une première dans laquelle la valve d'arrêt et la valve de fermeture sont toutes les deux ouvertes, une deuxième dans laquelle la valve d'arrêt est fermée et la valve de fermeture est ouverte, et une troisième dans laquelle les deux valves sont fermées, caractérisé en ce que le moyen mobile (20, 120) est déplaçable par rotation et/ou linéairement par rapport à la base, l'ensemble pouvant être ajusté dans les trois positions grâce à ce moyen.

11. Sac collecteur selon la revendication 10, caractérisé en ce que l'ensemble de valve dans sa troisième position, est positionné entièrement à l'intérieur de la périphérie des parois en feuille (1, 2).
